# DEMANDE DE BREVET EUROPEEN

(11) **EP 0 608 153 A1**
(43) Date de publication de la demande: **27.07.1994**
(21) Numéro de dépôt: 94400001.7
(22) Date de dépôt: 03.01.1994
(51) Int. Cl.: C12M 1/12, C12M 3/06, C12N 15/10

(54) **Appareil de dosage automatique et de réaction, en particulier pour l'extraction d'ADN**

(30) Priorité: 19.01.1993 FR 9300460
(71) Demandeur: BERTIN & CIE, F-78373 Plaisir Cedex (FR)
(72) Inventeur: Hache, Jean, F-78960 Voisin le Bretonneux (FR); Boquet, Jean, F-78610 Le Perray en Yvelines (FR); Ginot, Frédéric, F-78373 Plaisir (FR)
(74) Mandataire: Ramey, Daniel

(57) **Abrégé**

. Appareil de dosage automatique et de réaction, en particulier pour l'extraction d'ADN plasmidique d'un échantillon de cellules, comprenant une chambre de réaction (10) dont le fond muni d'un filtre comprend au moins un orifice de sortie (16), un piston (26) monté mobile à étanchéité dans la chambre (10), des moyens (32,34) d'amenée d'un échantillon de cellules entre le filtre (24) et le piston (26), et des moyens (36,38,40,42) de dosage et d'amenée de réactifs dans la chambre (10), l'introduction des réactifs étant autorisée quand le piston (26) est amené en position haute.

## Description

L'invention concerne un appareil de dosage automatique et de réaction, en particulier pour l'extraction d'ADN plasmidique à partir d'un échantillon de cellules, cet appareil étant du type comprenant une chambre de réaction dont le fond muni d'un filtre comprend au moins un orifice de sortie, des moyens d'obturation de cet orifice, un piston monté mobile de façon sensiblement étanche dans cette chambre au-dessus du filtre, des moyens de déplacement du piston, et des moyens pour amener des produits et des réactifs dans ladite chambre entre le fi Itre et le piston.

Un appareil de ce type est déjà connu, en particulier pour l'extraction d'ADN plasmidique, par les Demandes Internationales WO 90/15 148 et WO 89/09265.

Cependant, les appareils décrits dans ces deux Demandes Internationales présentent un certain nombre d'inconvénients tels que le risque d'obstruction complète du filtre par les cellules lors de la concentration parfiltrage de l'échantillon de cellules, l'utilisation d'un barreau agitateur au-dessus du filtre, ce qui crée un volume mort relativement important, et le dosage des réactifs par déplacement contrôlé du piston dans la chambre de réaction, d'où il résulte des erreurs relativement importantes sur les dosages.

La présente invention a notamment pour but d'éviter ces inconvénients dans un appareil du type précité.

Elle a pour objet un appareil de ce type, dont le fonctionnement peut être automatisé avec une très grande précision, notamment au niveau du dosage des réactifs.

Elle a également pour objet un appareil de ce type, ayant un rendement maximal en produit recherché.

Elle propose à cet effet un appareil de dosage automatique et de réaction du type précité, caractérisé en ce que des moyens de dosage de réactifs extérieurs à la chambre de réaction sont reliés à celle-ci par des conduits débouchant au-dessus d'un niveau maximal prédéterminé de liquide dans ladite chambre, et en ce que le piston est déplaçable entre une position où il est sensiblement appliqué sur le filtre, une position intermédiaire correspondant à un volume déterminé réglable au-dessus du filtre, et une position haute où il se trouve au-dessus des débouchés des conduits précités d'introduction de réactifs.

Dans l'appareil selon l'invention, les dosages de réactifs peuvent donc être réalisés à l'extérieur de la chambre, de façon extrêmement précise, le piston mobile dans la chambre de réaction étant utilisé, non pas pour le dosage proprement dit d'un réactif, mais uniquement pour permettre l'introduction d'une quantité déjà dosée d'un réactif dans la chambre, lorsqu'il se trouve au-dessus du débouché du conduit d'introduction correspondant.

Les dosages des réactifs sont ainsi totalement indépendants des déplacements du piston dans la chambre de réaction.

En fait, le déplacement du piston dans sa position haute pour l'introduction d'un réactif dans la chambre de réaction, se traduit par l'établissement d'une dépression dans cette chambre, qui facilite l'amenée dans cette chambre de la totalité d'une quantité pré- dosée de réactif.

Selon une autre caractéristique de l'invention, les moyens d'amenée de produit, en particulier d'un échantillon de cellules, comprennent un conduit d'injection débouchant tangentiellement dans la chambre de réaction immédiatement au-dessus du filtre précité, et des moyens d'alimentation sous pression de ce conduit.

Le produit qui est ainsi injecté dans la chambre de réaction préalablement remplie d'eau pure circule en tourbillonnant dans la partie inférieure de cette chambre, juste au-dessus du filtre, ce qui permet d'obtenir une concentration de ce produit par écoulement d'un milieu liquide à travers le filtre, sans risquer d'obstruer ce filtre par un dépôt de cellules par exemple, grâce à la circulation tourbillonnaire du produit dans la chambre de réaction.

En outre, le produit est injecté dans un volume faible au-dessus du filtre, et l'on n'a pas à réduire ce volume par descente du piston pour concentrer le produit.

Selon encore une autre caractéristique de l'invention, cet appareil comprend plusieurs moyens de dosage de réactifs, agencés autour de la chambre de réaction et comprenant chacun une chambre de dosage reliée à un réservoir de réactif, un moyen de réglage de la quantité de réactif admise dans la chambre de dosage, et un conduit reliant la chambre de dosage à la chambre de réaction.

Ce conduit peut comprendre un moyen d'obturation commandé, tel qu'une électrovanne par exemple.

Le piston de la chambre de réaction peut également comprendre une surface cylindrique périphérique d'obturation à étanchéité des débouchés des conduits d'introduction de réactifs, permettant l'ouverture de ces conduits uniquement quand le piston est au voisinage immédiat de sa position haute.

De préférence, les réservoirs de réactifs sont sous pression, pour garantir un remplissage correct des chambres de dosage.

L'appareil selon l'invention comprend également des moyens d'alimentation en liquide de lavage et de décontamination.

Enfin, la chambre de réaction peut être entourée d'une chemise de circulation d'un liquide de chauffage ou de refroidissement.

L'invention sera mieux comprise et d'autres caractéristiques, détails et avantages de celle-ci apparaîtront plus clairement à la lecture de la description qui suit, faite à titre d'exemple en référence aux dessins annexés dans lesquels :
La figure 1 représente schématiquement un appareil selon l'invention ;
La figure 2 est une vue schématique en section transversale, représentant une alimentation tangentielle de cette chambre ;
La figure 3 est un graphe représentant les différentes positions du piston de la chambre de réaction en fonction du temps ;
La figure 4 est un organigramme schématique des principales étapes d'un procédé d'extraction d'ADN au moyen de l'appareil selon l'invention.

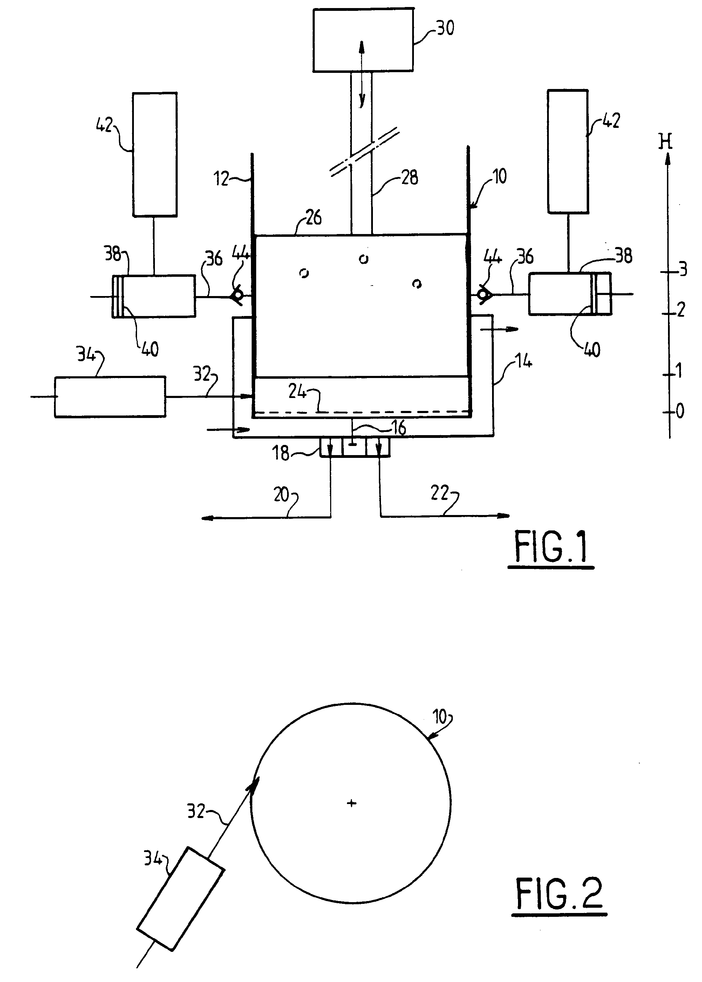

Cet appareil, dont un mode de réalisation est représenté à titre d'exemple en figure 1, comprend essentiellement une chambre de réaction 10 à paroi cylindrique 12, qui est entourée d'une chemise 14 de circulation d'un liquide caloporteur, par exemple d'eau, à température réglable, pour amener la température dans la chambre de réaction 10 à une valeur désirée.

Le fond de la chambre 10 comprend un ou plusieurs conduits de sortie 16 et un moyen 18 d'obturation commandée, tel qu'une électrovanne à boisseau ou à pointeau, ou une vanne à tiroir.

Dans l'exemple de réalisation représenté, le fond de la chambre 10 comprend un seul conduit de sortie 16 raccordé par une vanne à tiroir à deux conduits 20,22, dont l'un est utilisé pour la sortie de certains produits de réaction et dont l'autre est utilisé pour la sortie d'autres produits de réaction, comme on le verra plus en détail ci-dessous.

Immédiatement au-dessus de l'orifice supérieur du conduit de sortie 16, la chambre 10 comprend un filtre 24, tel par exemple qu'une membrane micropo- reuse, qui laisse passer certains produits de réaction et en retient d'autres. Le filtre 24 est disposé sur le fond de la chambre 10 de telle sorte que le volume résiduel entre le filtre et le fond soit le plus faible possible.

Un piston cylindrique 26 est monté coulissant à étanchéité dans la chambre 10, et est déplaçable de façon commandée au moyen d'une tige de piston 28 s'étendant vers le haut et d'un moteur 30 de déplacement rectiligne alternatif de la tige de piston 28 et du piston 26.

Le piston 26 peut occuper plusieurs positions déterminées réglables dans la chambre de réaction 10, qui sont définies par les références 0, 1, 2, 3 sur l'axe H de la droite de la figure 1, ces positions correspondant à celles de la face inférieure du piston.

Dans la position 0, la face inférieure du piston 26 se trouve sensiblement appliquée sur le filtre 24 et est donc au voisinage immédiat du fond de la chambre de réaction 10, le volume résiduel étant sensiblement nul.

Dans la position 1, la face inférieure du piston se trouve au-dessus du filtre 24, à faible distance de celui-ci, et détermine un volume prédéterminé avec le fond de la chambre de réaction. Dans ce volume prédéterminé débouche un conduit 32 d'injection de produit, ce conduit 32 étant orienté de façon sensiblement tangentielle à la chambre de réaction 10 comme on le voit en figure 2. Il est relié à un moyen 34 d'alimentation sous pression.

Dans la position 2, la face inférieure du piston 26 se trouve au-dessus du niveau maximal de liquide dans la chambre de réaction et en dessous des débouchés de conduits 36 d'introduction de réactifs, qui sont reliés à des moyens de dosage comprenant essentiellement une chambre de dosage 38, un moyen, tel qu'un piston mobile 40, de réglage de la quantité de réactif admise dans la chambre 38, et un réservoir 42 de réactif, de préférence sous faible pression, qui est relié à la chambre de dosage 38. Des moyens d'obturation commandés, tels que des électrovannes, sont en général prévus entre le réservoir 42 et la chambre 38, et sur les conduits d'introduction 36 au voisinage des débouchés de ces derniers dans la chambre de réaction 10. Des clapets anti-retour44 peuvent également être prévus sur les conduits d'introduction 36, comme représenté au dessin.

La position 2 précitée et la position 3 de la face intérieure du piston sont déterminées de telle sorte que les débouchés des conduits d'introduction 36 soient obturés de façon sensiblement étanche par la paroi cylindrique du piston 26 quand celui-ci est en position 2, et soient libérés quand le piston est en position 3, où sa face inférieure est légèrement au-dessus des débouchés de ces conduits 36.

Le nombre de systèmes de dosage et d'introduction de réactifs dans la chambre 10 est variable en fonction du type de réaction à réaliser. Les débouchés des conduits 36 d'introduction des réactifs peuvent être répartis à la même hauteur au-dessus du fond de la chambre 10 ou bien être étagés en hauteur de façon à pouvoir être libérés successivement lors du déplacement du piston 26 vers le haut. Dans ce cas, on peut prévoir autant de positions supérieures 3, 3',... du piston qu'il y a de niveaux de débouchés de conduits 36.

Au moins un réservoir de liquide de lavage et de décontamination est agencé au voisinage de la chambre 10. Il peut être relié à celle-ci par un conduit d'introduction 36 comme les réservoirs de réactifs 42, ou bien par l'un des conduits de sortie 20, 22 si l'on désire faire circuler ce liquide à travers le filtre 24 pour le nettoyage de ce dernier.

On va maintenant décrire le fonctionnement de cet appareil, en particulier dans le cas de l'extraction de l'ADN plasmidique d'un échantillon de cellules.

L'appareil ayant été préalablement lavé, décontaminé et rempli d'eau pure et le piston étant amené en position 1, un échantillon de cellules dans un liquide de culture est amené dans le moyen 34 et injecté sous pression par le conduit 32 dans la chambre 10 entre le filtre 24 et le piston 26. Dans le cas de l'extraction de l'ADN plasmidique, le volume au-dessus du filtre peut être de l'ordre de 0,5 ml tandis que l'échantillon de cellules à injecter est plusieurs fois supérieur (par exemple de l'ordre de 2 à 4 ml). La vanne à tiroir 18 est commandée pour relier le conduit de sortie 16 au conduit 20, parexemple. Dans ces conditions, l'injection progressive de l'échantillon de cellules dans la chambre de réaction 10 se traduit par un mouvement tourbillonnaire dans la chambre 10 et par un écoulement du liquide de culture à travers le filtre 24, les cellules restant en mouvement au-dessus de ce filtre sans le colmater.

Il en résulte simultanément une concentration de cet échantillon, sans qu'il soit nécessaire de déplacer le piston 26 qui est maintenu dans sa position 1.

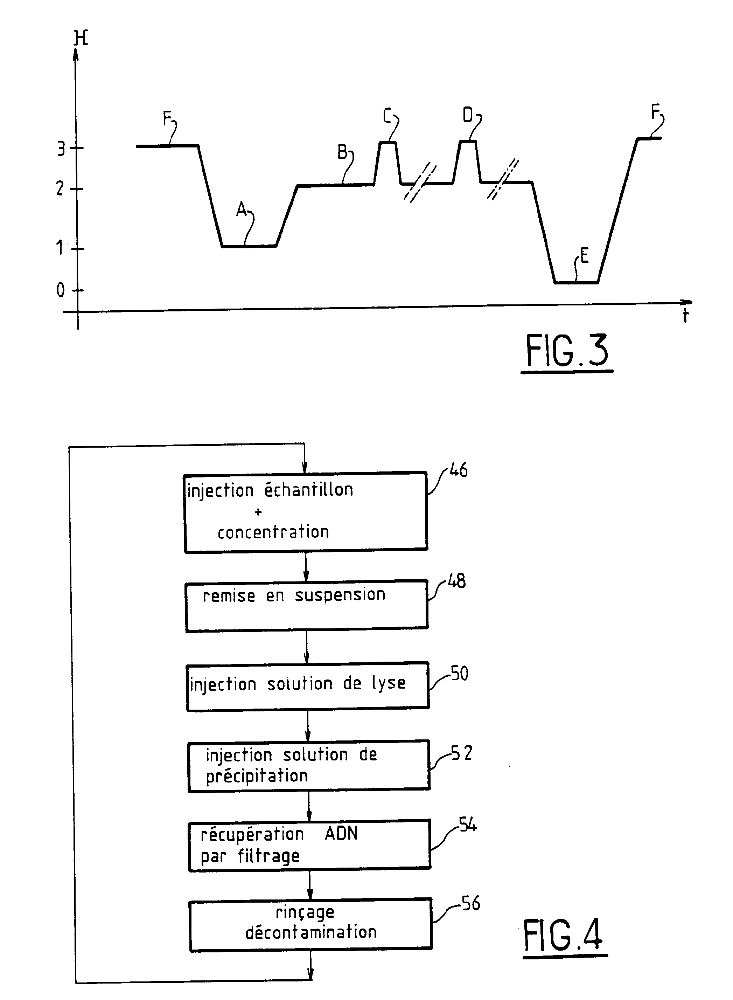

Cette étape correspond à la partie A de la courbe de la figure 3 et est désignée par la référence 46 en figure 4.

L'opération suivante consiste à fermer le conduit de sortie 16 par commande de la vanne 18 et à obturer le conduit d'injection 32, après quoi on amène le piston 26 dans sa position 2 pour réaliser une remise en suspension des cellules. Le déplacement du piston 26 de sa position 1 à sa position 2 se traduit par une dépression dans la chambre de réaction 10, qui facilite la remise en suspension des cellules et par une faible vaporisation du liquide, suivie d'une recon- densation quand le piston est ramené en position 1, de sorte que la face inférieure du piston est toujours en contact intime avec du liquide en position 1, sans interposition d'une couche de gaz entre le piston et le liquide. On peut aussi appliquer une faible vibration à la chambre de réaction 10, ou bien la faire basculer autour d'un axe transversal horizontal pourfaciliter la remise en suspension des cellules.

Cette étape est désignée par la référence B sur la courbe de la figure 3 et par la référence 48 dans l'organigramme de la figure 4. A la fin de cette étape, le piston peut être en position 1 ou en position 2.

Pendant ce temps, deux systèmes de dosage de réactifs ont été utilisés, l'un pour le dosage d'une solution de lyse des cellules, l'autre pour le dosage d'une solution de précipitation de l'ADN chromosomique, des protéines et des débris de cellules. Pour ces dosages, les moyens d'obturation des conduits 36 sont fermés, les moyens d'obturation prévus entre les réservoirs 42 et les chambres 38 sont ouverts, et les pistons 40 sont déplacés dans des positions prédéterminées pour l'admission de quantités prédéterminées de produits dans les chambres 38.

Dans le cas de l'extraction de l'ADN plasmidique d'un échantillon de cellules, on admet d'abord un volume de l'ordre de 0,5 ml d'une solution de lyse des cellules dans la chambre 10 (partie C de la courbe de la figure 3 et étape 50 de l'organigramme de la figure 4), après quoi on ramène le piston 26 dans sa position 2 pendant la lyse des cellules. Simultanément, la chambre de réaction peut être portée à une température de l'ordre de 50° C par circulation d'eau chaude dans la chemise 14. Au bout de quelques minutes, on interrompt cette circulation d'eau chaude, et on ramène le piston 26 en position 3 pour l'admission d'un volume de l'ordre de 0,5 ml d'une solution de précipitation de l'ADN chromosomique, des protéines et des débris de cellules, laissant l'ADN plasmidique en solution (partie D de la courbe de la figure 3 et étape 52 de l'organigramme de la figure 4).

Le piston 26 est ensuite amené en position 0 (partie E de la courbe de la figure 3 et étape 54 de l'organigramme de la figure 4), la vanne 18 étant commandée pour relier l'orifice de sortie 16 au conduit 22, afin d'évacuer le contenu de la chambre 10 par le conduit 22 et récupérer ainsi l'ADN plasmidique en solution, tandis que l'ADN chromosomique précipité, les protéines et les débris de cellules sont retenus par le filtre 24.

Il est important que ce filtre soit le plus près possible du fond de la chambre de réaction 10, pour que l'on puisse récupérer la plus grande quantité possible d'ADN plasmidique. L'appareil selon l'invention permet de disposer le filtre 24 au voisinage immédiat du fond de la chambre de réaction 10, et de descendre le piston 26 jusque sur le filtre 24.

L'étape suivante, désignée par la référence 56 en figure 4 et correspondant à la partie F de la courbe de la figure 3, consiste à effectuer un lavage et une décontamination de la chambre 10, du conduit 32 d'injection de l'échantillon de cellules et éventuellement des conduits 20 et 22 de sortie de la chambre 10. Pour cela, on peut introduire un liquide de lavage et de décontamination dans la chambre 10 au moyen d'un système identique aux systèmes de dosage des réactifs, ou bien par l'intermédiaire du conduit de sortie 20, ce qui permet alors de nettoyer également le filtre 24 par circulation de liquide vers le haut à travers ce filtre. On utilise avantageusement le conduit 32 pour évacuer de la chambre 10 le liquide de lavage et de décontamination, ainsi que les divers produits qu'il peut contenir (ADN précipité, protéines, débris de cellules). On peut également effectuer, avant le lavage et la décontamination, une opération préalable de dissolution de ces produits par injection d'une solution de dissolution dans la chambre 10 au moyen d'un système identique aux systèmes précités de dosage de réactifs.

De façon générale, l'appareil selon l'invention présente les avantages suivants :
- le volume mort entre la face inférieure du piston dans sa position basse et le fond de la chambre de réaction 10 est sensiblement nul,
- on récupère une quantité maximale de produit recherché (ADN plasmidique),
- les dosages de réactifs sont complètement indépendants des déplacements du piston 26 dans la chambre 10,
- l'injection tangentielle de l'échantillon de cellules permet simultanément de concentrer cet échantillon sans colmater ou obstruer le filtre 24 et sans déplacer le piston 26,
- la commande de déplacement du piston 26 est plus douce et est soumise à moins de contraintes que dans la technique antérieure,
- le barreau magnétique prévu dans la technique antérieure pour servir d'agitateur entre le filtre 24 et la face inférieure du piston 26 est supprimé.

Bien entendu, l'appareil selon l'invention est applicable à un très grand nombre de réactions différentes, et n'est pas limité à l'extraction d'ADN plasmidique.

## Revendications

1. Appareil de dosage automatique et de réaction, en particulier pour l'extraction d'ADN plasmidique d'un échantillon de cellules, comprenant une chambre de réaction (10) dont le fond muni d'un filtre (24) comprend au moins un orifice de sortie (16), des moyens (18) d'obturation de cet orifice, un piston (26) monté mobile de façon sensiblement étanche dans cette chambre au-dessus du filtre (24), des moyens (28,30) de déplacement du piston, des moyens (32,34,36) pour amener des produits et des réactifs dans ladite chambre entre le filtre (24) et le piston (26), caractérisé en ce que des moyens (38,40,42) de dosage de réactifs extérieurs à la chambre (10) sont reliés à celle-ci par des conduits (36) débouchant au-dessus d'un niveau maximal prédéterminé de liquide dans ladite chambre, et en ce que le piston (26) est déplaçable entre une position où il est sensiblement appliqué sur le filtre (24), une position intermédiaire correspondant à un volume déterminé réglable au-dessus du filtre, et une position haute où il se trouve au-dessus des débouchés des conduits (36) d'introduction de réactifs.

2. Appareil selon la revendication 1, caractérisé en ce que les moyens d'amenée de produit, tel en particulier qu'un échantillon de cellules, comprennent un conduit d'injection (32) débouchant tangentiellement dans la chambre de réaction (10) immédiatement au-dessus du filtre (24), et des moyens (34) d'alimentation sous pression de ce conduit.

3. Appareil selon la revendication 1 ou 2, caractérisé en ce qu'il comprend plusieurs moyens de dosage de réactifs, agencés autour de la chambre de réaction (10) et comprenant chacun une chambre (38) de dosage reliée à un réservoir(42) de réactif, un moyen (40) de réglage de la quantité de réactif admise dans la chambre de dosage (38), et un conduit (36) reliant la chambre de dosage à la chambre de réaction (10).

4. Appareil selon la revendication 3, caractérisé en ce que le conduit (36) reliant la chambre de dosage (38) à la chambre de réaction (10) comprend un moyen d'obturation commandé, tel qu'une électrovanne par exemple, et/ou un clapet anti-retour (44).

5. Appareil selon la revendication 3 ou 4, caractérisé en ce que le piston (26) comprend une surface périphérique d'obturation à étanchéité des débouchés des conduits (36) d'introduction de réactifs, permettant l'ouverture de ces débouchés uniquement quand le piston (26) est au voisinage immédiat de sa position haute.

6. Appareil selon l'une des revendications 3 à 5, caractérisé en ce que les réservoirs (42) de réactifs sont sous pression.

7. Appareil selon l'une des revendications précédentes, caractérisé en ce qu'il comprend également des moyens d'alimentation en liquide de lavage et de décontamination.

8. Appareil selon l'une des revendications précédentes, caractérisé en ce que la chambre de réaction (10) est entourée d'une chemise (14) de circulation d'un liquide de chauffage ou de refroidissement.

9. Appareil selon une des revendications précédentes, caractérisé en ce que les débouchés dans la chambre (10) des conduits (36) d'amenée de réactifs sont à un même niveau, ou à des niveaux différents.

10. Appareil selon l'une des revendications précédentes, caractérisé en ce que le déplacement du piston (16) de sa position intermédiaire (1) à sa position (2) sous les débouchés des conduits (36) d'amenée de réactifs, se traduit par une dépression dans la chambre (10) et par une vaporisation partielle du liquide qu'elle contient.
